**Europäisches Patentamt**

**European Patent Office**

**Office européen des brevets**

(19)

(11) **EP 1 398 308 A1**

(12) **EUROPÄISCHE PATENTANMELDUNG**

(43) Veröffentlichungstag:
**17.03.2004 Patentblatt 2004/12**

(51) Int Cl.⁷: **C07C 51/373**, C07C 59/185

(21) Anmeldenummer: **03017237.3**

(22) Anmeldetag: **30.07.2003**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **12.08.2002 DE 10236919**

(71) Anmelder: **Bayer Chemicals AG**
**51368 Leverkusen (DE)**

(72) Erfinder: **Fiege, Helmut, Dr.**
**51373 Leverkusen (DE)**

(54) **Verfahren zur Herstellung von 3,3 Dimethyl-2-oxobuttersäure**

(57) Die Erfindung betrifft ein Verfahren zur Herstellung von 3,3-Dimethyl-2-oxobuttersäure und ihren Salzen durch Oxidation von 3,3-Dimethyl-2-hydroxybuttersäure mit Sauerstoff oder Sauerstoff enthaltenden Gasen in basisch-wässrigem Medium und in Gegenwart von Palladiumkatalysatoren sowie Bismut oder Bismutverbindungen.

## Beschreibung

Die Erfindung betrifft ein Verfahren zur Herstellung von 3,3-Dimethyl-2-oxobuttersäure und ihren Salzen durch Oxidation von 3,3-Dimethyl-2-hydroxybuttersäure mit Sauerstoff oder Sauerstoff enthaltenden Gasen in basisch-wässrigem Medium und in Gegenwart von Palladiumkatalysatoren sowie Bismut oder Bismutverbindungen.

3,3-Dimethyl-2-oxobuttersäure wird, speziell in Form ihrer wässrigen Natriumsalzlösung, zur großtechnischen Herstellung von Agrochemikalien wie insbesondere des vornehmlich in Kulturen von Sojabohnen, Tomaten und Kartoffeln eingesetzten selektiven Herbizids Metribuzin (Sencor®) benötigt (siehe auch US-Patent 3,671,523 und US-Patent 3,905,801).

Aus EP-A 1 097 917 ist eine Flüssigphasen-Oxidation von 2-Hydroxycarbonsäuren oder deren Estern mit hypobromiger Säure bekannt. Für den großtechnischen Einsatz ist jedoch der Einsatz von hypobromiger Säure als Oxidationsmittel aus ökologischen und wirtschaftlichen Gründen nicht vertretbar.

Aus EP-A 0 011 207 ist ein Verfahren zur Herstellung von 3,3-Dimethyl-2-oxobuttersäure und/oder ihren Salzen bekannt, das über die Oxidation von 3,3-Dimethyl-2-hydroxybuttersäure in wässrig-alkalischer Lösung mit unterchloriger Säure (Hypochlorit) in Gegenwart eines Rutheniumkatalysators verläuft. Allerdings beeinträchtigen auch hier der Preis und die geringe Umweltverträglichkeit des Oxidationsmittels die industrielle Anwendung.

Ein ähnliches Verfahren, das im US-Patent 5,091,568 beschrieben ist, umgeht diese Nachteile durch Verwendung von Sauerstoff als Oxidans. Jedoch werden hierbei deutlich höhere Katalysatormengen von bis zu 5 Gew.-% und ein hoher Sauerstoffdruck von 20 bis 40 bar benötigt, was das Verfahren ineffizient werden lässt.

Weiterhin ist aus DE-OS 29 15 395 bekannt, dass Natrium-2-hydroxypropionat mit Sauerstoff oder Luft an einem Platin-Katalysator bei Gegenwart von Bismut oder Blei oder seinen Verbindungen bei 35-70°C mit Ausbeuten von 80-90 % zu Natrium-2-oxopropionat oxidiert werden kann. Die Anwendung auf die Oxidation von 3,3-Dimethyl-2-hydroxy-buttersäure bzw. deren Natriumsalz ist nicht beschrieben. Eigene Untersuchungen ergaben jedoch, dass das beschriebene Verfahren dafür völlig ungeeignet ist (siehe Vergleichsbeispiele).

Es bestand daher das Bedürfnis, ein Verfahren zu entwickeln, das die Herstellung von 3,3-Dimethyl-2-oxobuttersäure bzw. deren Salzen in effizienter Weise ermöglicht.

Es wurde nun ein Verfahren zur Herstellung von 3,3-Dimethyl-2-oxobuttersäure bzw. deren Salzen durch übergangsmetallkatalysierte Oxidation von 3,3-Dimethyl-2-hydroxybuttersäure gefunden, das dadurch gekennzeichnet ist, dass es in Gegenwart

- von Sauerstoff,
- Palladiumkatalysator,
- Bismut und/oder Bismutverbindungen,
- Wasser und
- Base

durchgeführt wird.

Im Rahmen der Erfindung können alle aufgeführten allgemeinen oder in Vorzugsbereichen aufgeführten Parameter bzw. Erläuterungen untereinander, also auch zwischen den jeweiligen Bereichen und Vorzugsbereichen beliebig kombiniert werden.

Die 3,3-Dimethyl-2-hydroxybuttersäure kann im erfindungsgemäßen Verfahren in der D-, der L-Form oder in beliebigen Mischungen der Enantiomeren wie insbesondere der racemischen Form eingesetzt werden. Weiterhin kann 3,3-Dimethyl-2-hydroxybuttersäure auch zumindest teilweise in Form eines Salzes wie insbesondere eines Alkalimetallsalzes eingesetzt werden.

3,3-Dimethyl-2-hydroxybuttersäure kann nach bekannten Verfahren, beispielsweise durch Umsetzung von Dichlorpinakolin mit wässrigem Alkali hergestellt werden (siehe auch DE-OS 26 48 300). Die dabei anfallende Alkalisalzlösung der 3,3-Dimethyl-2-hydroxybuttersäure kann beispielsweise direkt für das erfindungsgemäße Verfahren eingesetzt werden.

Bei der Durchführung des erfindungsgemäßen Verfahrens entsteht die 3,3-Dimethyl-2-oxobuttersäure überwiegend in Form ihrer Salze, aus denen die freie Säure gegebenenfalls durch Ansäuern gewonnen werden kann. Die erhaltene Reaktionslösung kann aber gegebenenfalls auch direkt für weitere Umsetzungen wie z.B. mit Thiocarbohydrazid zur Bildung von 4-Amino-6-tert.-butyl-3-thio-1,2,4-triazin-5(4H)-on, verwendet werden.

Das erfindungsgemäße Verfahren wird in Gegenwart von Palladiumkatalysator durchgeführt. Als Palladiumkatalysator eignet sich beispielsweise metallisches Palladium wie insbesondere Palladium-Mohr oder auf einen Träger aufgebrachtes Palladium. Als Träger geeignet sind beispielsweise Aktivkohle, Graphit, Kieselgur, Kieselgel, Spinelle, Aluminiumoxide, Calciumcarbonat, Magnesiumoxid, Bariumsulfat oder auch organische Trägermaterialien.

Bevorzugt werden als Trägermaterialien pulverförmige Aktivkohlen, beispielsweise sogenannte Medizinalkohlen oder aus Holz hergestellte Aktivkohlen, wie sie vielfach für Entfärbungszwecke verwendet werden, eingesetzt.

Der Palladium-Gehalt der Trägerkatalysatoren ist unkritisch und kann innerhalb eines großen Bereichs variiert werden. Im Allgemeinen verwendet man Trägerkatalysatoren, deren Gehalt an Palladium zwischen 0,1 und 20 Gew.-%, vorzugsweise zwischen 0,5 und 15 Gew.-% liegt.

Auch die Menge, in der der Palladium-Katalysator eingesetzt wird, kann in einem größeren Bereich variiert werden. Sie hängt unter anderem von der gewünschten Oxidationsgeschwindigkeit ab. Im Allgemeinen wählt man die Katalysatormenge so, dass das molare Verhältnis von Palladium zu eingesetzter 3,3-Dimethyl-2-hydroxybuttersäure bzw. deren Salzen zwischen 1:5 und 1:20.000, bevorzugt zwischen 1:10 und 1:10.000 und besonders bevorzugt zwischen 1:100 und 1:10.000 liegt.

Die Aktivität und Selektivität des Palladium-Katalysators wird bei dem erfindungsgemäßen Verfahren durch die Anwesenheit von Bismut und/oder dessen Bismutverbindungen erheblich gesteigert, was sich auch positiv auf die Wiederverwendbarkeit des Palladiumkatalysators auswirkt.

Die Menge, in der Bismut und/oder dessen Bismutverbindungen eingesetzt werden, kann in weiten Grenzen variiert werden. So kann das molare Verhältnis von Bismut oder Bismutverbindung zu eingesetzter 3,3-Dimethyl-2-hydroxybuttersäure oder deren Salze beispielsweise $1 \cdot 10^{-6}$ bis $1 \cdot 10^{-1}$, bevorzugt $5 \cdot 10^{-6}$ bis $1 \cdot 10^{-1}$ und besonders bevorzugt $2 \cdot 10^{-5}$ bis $2 \cdot 10^{-2}$ betragen. Größere Mengen sind möglich, aber unwirtschaftlich.

Bismut kann beispielsweise in elementarer Form und/oder in Form von Bismutverbindungen, z.B. als Oxid, Hydroxid, Oxidhydrat oder Salz von Wasserstoffsäuren wie Chlorid, Bromid, Jodid, Sulfid oder als Salz von anorganischen Sauerstoffsäuren wie Nitrat, Nitrit, Phosphit, Phosphat, Carbonat, Perchlorat oder als Salz von Sauerstoffsäuren, die von Übergangsmetallen abstammen, oder als Salz organischer Säuren oder als Komplexverbindung oder als metallorganische Verbindung eingesetzt werden.

Es ist auch möglich, Bismut und/oder Bismutverbindungen in Kombination mit anderen Metallen, Halbmetallen oder deren Verbindungen in das erfindungsgemäße Verfahren einzubringen.

Der erfindungsgemäß einzusetzende Bismut-Aktivator kann in unterschiedlichen und auch gemischten Wertigkeitsstufen vorliegen. Es können auch Änderungen in der Wertigkeit während der Reaktion eintreten. Ferner ist es möglich, dass sich das Bismut-und/oder die Bismutverbindungen im Reaktionsmedium ganz oder teilweise in andere Verbindungen umwandeln.

Das Bismut und/oder die Bismutverbindungen können als Feststoff, vorzugsweise in feinverteilter Form den Reaktionskomponenten zugesetzt werden. Man kann das Bismut und/oder die Bismutverbindungen aber auch schon bei der Herstellung des Palladium-Katalysators zugeben oder den Palladium-Katalysator mit Bismut und/oder den Bismutverbindungen imprägnieren. Bismut und/oder die Bismutverbindungen können auch als Trägermaterial für das Palladium dienen.

Die Konzentration der organischen Verbindungen (3,3-Dimethyl-2-hydroxybuttersäure und deren Reaktionsprodukte) in der Reaktionslösung wird vorzugsweise so gewählt, dass sowohl die 3,3-Dimethyl-2-hydroxybuttersäure als auch die gebildete 3,3-Dimethyl-2-oxobuttersäure unter den Reaktionsbedingungen überwiegend oder vorzugsweise vollständig gelöst vorliegen. Gegebenenfalls kann die 3,3-Dimethyl-2-hydroxybuttersäure dem Oxidationsgemisch - gegebenenfalls zusammen mit Base-nach und nach (kontinuierlich oder portionsweise) zugeführt werden. Bewährt haben sich Konzentrationen an organischen Verbindungen von 5 bis 30 Gew.-%. Niedrigere Konzentrationen z.B. sind auch möglich, aber weniger wirtschaftlich.

Das erfindungsgemäße Verfahren wird in Gegenwart von Wasser und Base durchgerührt. Vorzugsweise wird dabei die Base so bemessen, dass auf ein Mol zu oxidierende 3,3-Dimethyl-2-hydroxybuttersäure insgesamt 1,1 bis 8, vorzugsweise 1,2 bis 6 Moläquivalente Base eingesetzt werden. Wird die 3,3-Dimethyl-2-hydroxybuttersäure bereits als Alkalisalz bzw. in Form ihrer wässrigen Lösung eingesetzt, so sind die der Säure äquivalenten und in der Lösung evtl. bereits vorhandenen Basemengen bei diesen Vorgaben zu berücksichtigen. Größere Mengen Base sind prinzipiell möglich, aber unwirtschaftlich.

Als Base werden bevorzugt Alkali- und Erdalkali-carbonate sowie -hydroxide, Mischungen davon oder die entsprechenden wässrigen Lösungen eingesetzt. Besonders bevorzugt werden Natriumhydroxid und Kaliumhydroxid, Mischungen davon oder die entsprechenden wässrigen Lösungen eingesetzt.

Die Reaktion kann beispielsweise bei einem Druck von 0,1 bis 50 bar durchgeführt werden, bevorzugt bei 0,5 bis 5 bar, besonders bevorzugt bei 0,9 bis 1,5 bar und ganz besonders bevorzugt bei Umgebungsdruck.

Die Umsetzung erfolgt bevorzugt zwischen 20 und 150°C.

Vorteilhafterweise wird eine Temperatur im Bereich von 60°C bis zum Siedepunkt des Reaktionsgemisches beim gewählten Reaktionsdruck gewählt.

In einer besonders bevorzugten Ausführungsform des erfindungsgemäßen Verfahrens wird die untere Temperaturgrenze t (untere) so gewählt, dass sie der Formel

$$t \text{ (untere)} = 90 - n^2 \qquad (°C)$$

gehorcht, in der

n  die Äquivalente Alkali pro mol eingesetzter 3,3-Dimethyl-2-hydroxybuttersäure (abgekürzt: DHBS) bedeutet.

Es kann also zum Beispiel beim Einsatz von 5 mol NaOH/mol DHBS beispielsweise und bevorzugt im Bereich von etwa 65°C bis zum Siedepunkt, beim Einsatz von 3 mol NaOH/mol DHBS beispielsweise und bevorzugt im Bereich von etwa 81°C bis zum Siedepunkt, beim Einsatz von 2 mol NaOH/mol DHBS beispielsweise und bevorzugt im Bereich von etwa 86°C bis zum Siedepunkt und beim Einsatz von 1,5 mol NaOH/mol DHBS beispielsweise und bevorzugt im Bereich von etwa 88°C bis zum Siedepunkt des Reaktionsgemisches gearbeitet werden.

Ganz besonders bevorzugt wird im Bereich von 90°C bis zum Siedepunkt gearbeitet, da in diesem Temperaturbereich sowohl bei kleinen als auch bei größeren Alkali/-DHBS-Molverhältnissen hohe Ausbeuten in kurzer Zeit möglich sind und die hohen Temperaturen reaktionstechnisch zugleich eine gute Abfuhr der Reaktionswärme der exotherm verlaufenden Oxidation ermöglichen.

Die Reihenfolge, in der Palladium-Katalysator, Bismut und/oder Bismutverbindungen, Wasser, Base und 3,3-Dimethyl-2-hydroxybuttersäure zusammengegeben werden, ist beliebig und unkritisch. So können Palladium-Katalysator und Bismut und/oder Bismutverbindungen der Mischung oder Lösung aus Wasser, Base und 3,3-Dimethyl-2-hydroxybuttersäure zugesetzt werden. Man kann auch den Palladium-Katalysator und Bismut und/oder Bismutverbindungen vorlegen und die Mischung oder Lösung aus Wasser, Base und 3,3-Dimethyl-2-hydroxybuttersäure zusetzen. Schließlich ist es auch möglich, den Palladium-Katalysator, einen Teil des Wasser-Base-Gemischs sowie Bismut und/oder Bismutverbindungen vorzulegen und die 3,3-Dimethyl-2-hydroxybuttersäure dann, gegebenenfalls zusammen mit der restlichen Base oder restlicher Mischung aus Wasser und Base, hinzuzusetzen. Ferner ist es möglich, Bismut und/oder Bismutverbindungen der Mischung der übrigen Komponenten zuzusetzen.

Im Allgemeinen wird das erfindungsgemäße Verfahren so durchgeführt, dass man Sauerstoff oder Sauerstoff enthaltende Gase, wie beispielsweise Luft, mit dem Reaktionsgemisch, das Wasser, Base, den Palladium-Katalysator, Bismut und/oder Bismutverbindungen sowie die 3,3-Dimethyl-2-hydroxybuttersäure enthält, in intensiven Kontakt bringt.

Der Katalysator braucht im Reaktionsgemisch nicht als Pulver suspendiert vorzuliegen, sondern kann auch in gekörnter Form als Festbett angeordnet sein, das von den übrigen Komponenten durchströmt wird.

Der Verlauf der Reaktion kann zum Beispiel über die Messung der aufgenommen Sauerstoffmenge verfolgt werden. Die Umsetzung kann abgebrochen werden, wenn die zur Erzielung einer optimalen Selektivität erforderliche Sauerstoffmenge aufgenommen ist. Zur Erzielung hoher Selektivitäten bricht man die Reaktion vorzugsweise bei einer Sauerstoffaufnahme von 0,2 bis 0,5 mol Sauerstoff/mol 3,3-Dimethyl-2-hydroxybuttersäure ab, besonders bevorzugt nach 0,4 mol bis 0,5 mol Sauerstoff/mol 3,3-Dimethyl-2-hydroxybuttersäure. Will man eine gute Ausbeute erzielen, bricht man die Reaktion vorzugsweise nach Aufnahme von 0,4 bis 0,6 mol Sauerstoff/mol 3,3-Dimethyl-2-hydroxybuttersäure ab, besonders bevorzugt nach Aufnahme von 0,45 bis 0,58 mol Sauerstoff/mol 3,3-Dimethyl-2-hydroxybuttersäure und ganz besonders bevorzugt nach Aufnahme von 0,50 bis 0,56 mol Sauerstoff/mol 3,3-Dimethyl-2-hydroxybuttersäure. Welcher Wert zweckmäßig ist, hängt u.a. von den gewählten Reaktions- und Aufarbeitungsbedingungen, der gewünschten Produktreinheit usw. ab und kann im Einzelfall durch Vorversuche ermittelt werden.

Der Fortgang der Reaktion kann auch auf andere Weise kontrolliert werden, z.B. durch Bestimmung der verbrauchten 3,3-Dimethyl-2-hydroxybuttersäure, der gebildeten 3,3-Dimethyl-2-oxobuttersäure und/oder der sich daraus in einer Folgereaktion gegen Reaktionsende durch Überoxidation in geringer Menge bildenden Trimethylessigsäure.

Die Aufarbeitung kann bei dem erfindungsgemäßen Verfahren nach üblichen Methoden erfolgen. Im Allgemeinen wird der Palladium-Katalysator nebst ungelöstem und adsorbiertem Bismut und/oder Bismutverbindungen abgetrennt, beispielsweise durch Filtrieren. Die erhaltene Lösung, welche die 3,3-Dimethyl-2-oxobuttersäure überwiegend in Form ihrer Salze und in hoher Ausbeute enthält, kann, gegebenenfalls nach teilweiser oder vollständiger Neutralisation der überstöchiometrischen Base, als solche weiterverwendet werden. Man kann aus der Lösung jedoch auch durch Ansäuern mit einer Mineralsäure, wie z.B. Salz-, Schwefel- oder Phosphorsäure, die 3,3-Dimethyl-2-oxobuttersäure in Freiheit setzen und diese nach bekannten Verfahren, z.B. durch Extraktion mit einem in Wasser wenig löslichen organischen Solvens, wie z.B. Ether (z.B. Diethylether, Diisopropylether) oder Ketone (z.B. Methylisobutylketon), isolieren. Die Extraktion kann auch bei unterschiedlichen pH-Stufen unter gleichzeitiger Reinigung erfolgen (siehe auch US-Patent 6,274,766). Nach Abdestillation des Extraktionsmittels kann eine weitere Reinigung, sofern erforderlich, z.B. durch fraktionierende Destillation, gegebenenfalls im Vakuum, erfolgen. Es ist auch möglich, die 3,3-Dimethyl-2-oxobuttersäure aus der zunächst anfallenden Alkalisalz-Lösung an einem Kationenaustauscher freizusetzen, durch schonendes Eindampfen der erhaltenen Lösung zu isolieren und erforderlichenfalls durch fraktionierende Destillation weiter zu reinigen.

Die erfindungsgemäß herstellbare 3,3-Dimethyl-2-oxobuttersäure bzw. deren Salze eignen sich insbesondere zur Herstellung von Agrochemikalien, wie Metribuzin. Vorzugsweise eignet sie sich zur Herstellung von 4-Amino-6-tert.-

butyl-3-thio-1,2,4-triazin-5 (4H)-on.

Das erfindungsgemäße Verfahren zeichnet sich dadurch aus, dass als Oxidationsmittel Sauerstoff (Luft) bei niederem Druck verwendet werden kann, die Oxidation auch bei geringen Katalysatorkonzentrationen mit sehr hohen Ausbeuten verläuft und durch die hohen Umsatzraten auch hohe Raum-Zeit-Ausbeuten realisiert werden können.

**Beispiele**

**Beispiel 1**

In ein mit Rührer, Innenthermometer und Gaszuleitung versehenes, über einen Heizmantel temperierbares Reaktionsgefäß werden 1,0 g Aktivkohle (Medizinalkohle) mit 5 Gew.-% Palladiumgehalt, 0,024 g Bi $(NO_3)_3$ •5 $H_2O$, 100 ml 3N Natronlauge und 13,2g (0,1mol) 3,3-Dimethyl-2-hydroxybuttersäure (DHBS) eingebracht.

Nach Verdrängen der Luft aus dem Reaktionsgefäß durch Sauerstoff wird das Einsatzgemisch auf 95°C erwärmt und bei dieser Temperatur gerührt, bis nach ca. 2 Stunden 0,0535 mol Sauerstoff (ca. 1.300 ml bei Raumtemperatur und Umgebungsdruck) aufgenommen sind. Es werden dann die Sauerstoffzufuhr und der Rührer abgestellt.

Nach Abfiltrieren des Kontaktes (er kann wiederverwendet werden) wird das Filtrat bei Raumtemperatur mit 20 %iger Salzsäure auf pH 7,5 eingestellt, die Filtratmenge ermittelt und in einem aliquoten Anteil der Gehalt an 3,3-Dimethyl-2-oxobuttersäure durch Differential-Puls-Polarographie bestimmt (Grundelektrolyt: Acetatpufferlösung). Die Bestimmung erfolgte gegen eine 3,3-Dimethyl-2-oxobuttersäure-Lösung bekannten Gehaltes, die bei einer Wiederholungsmessung bei derselben Probe als interner Standard zugegeben wurde. Die Bestimmung ergab eine 3,3-Dimethyl-2-oxobuttersäure-Ausbeute von 11,7 g (90 % d. Th.).

Nach Ansäuern des Filtrats auf pH 1, mehrfacher Extraktion mit Ether und Abdampfen des Ethers ließen sich im Extraktionsrückstand neben 3,3-Dimethyl-2-oxobuttersäure ca. 0,7 g (7 % d. Th.) Trimethylessigsäure gaschromatographisch nachweisen.

**Beispiele 2 - 5 (Vergleichsbeispiele)**

Es werden wie in Beispiel 1 beschrieben 0,1 mol (13,2 g) 3,3-Dimethyl-2-hydroxybuttersäure (DHBS) in Natronlauge mit Sauerstoff unter Normaldruck an 1 g Edelmetall/Aktivkohle-Kontakt zu 3,3-Dimethyl-2-oxobuttersäure (DOBS) oxidiert.

In Tabelle 1 sind die Art des verwendeten Edelmetall/Kohle-Katalysators, die Art und Menge des Zusatzes, die angewandte Natronlaugemenge, die Oxidationstemperatur, die aufgenommene Sauerstoffmenge, die zur Aufnahme dieser Sauerstoffmenge erforderliche Zeit und die erzielte, polarographisch bestimmte DOBS-Ausbeute angegeben. Zum Vergleich sind die Werte des Beispiels 1 mit in der Tabelle 1 aufgeführt.

Tabelle 1

| Beispiel Nr. | 1 | 2 | 3 | 4 | 5 |
|---|---|---|---|---|---|
| **Katalysator** | 5%Pd/ / A-Kohle | 5% Pd / A-Kohle | 5% Pd / A-Kohle | 1% Pt / A-Kohle | 1% Pt / A-Kohle |
| **Zusatz** | $Bi(NO_3)_3$ | $Pb(NO_3)_2$ | ohne | $Pb(NO_3)_2$ | $Pb(NO_3)_2$ |
| [mol/mol DHBS] | $5 \cdot 10^{-4}$ | $1,3 \cdot 10^{-3}$ | --- | $5 \cdot 10^{-3}$ | $5 \cdot 10^{-3}$ |
| **Natronlauge** [mol/mol DHBS] | 3 | 3 | 3 | 4 | 2 |
| **Temperatur** [°C] | 95 | 95 | 95 | 95 | 70 |
| **$O_2$-Aufnahme** | | | | | |
| [mol/mol DHBS] | 0,535 | 0,090 | 0,090 | 0,005 | 0,015 |
| Zeit [Stunden] | 2 | 8 | 8 | 1 | 1 |
| Stillstand | nein | fast | fast | ja | ja |
| **DOBS - Ausb.** [%d.Th.] | 90 | 5 | 5 | < 1 (nich aufgearbeitet) | < 3 (nicht aufgearbeitet) |

### Beispiele 6 - 15

Es werden wie in Beispiel 1 beschrieben 0,1 mol (13,2 g) 3,3-Dimethyl-2-hydroxybuttersäure (DHBS) in 100 ml 5N Natronlauge (= 5 mol NaOH/mol DHBS) bei verschiedenen Temperaturen an 1 g Aktivkohle (Medizinalkohle) mit 5 Gew.-% Palladiumgehalt bei Gegenwart von $5 \cdot 10^{-4}$ mol $Bi(NO_3)_3$ /mol DHBS als Zusatz unter Normaldruck bis zur Aufnahme von ca. 1.300 ml Sauerstoff (ca. 0,535 mol $O_2$ /mol DHBS) oxidiert. Die Ergebnisse hinsichtlich Reaktionszeit und polarographisch bestimmter Ausbeute an 3,3-Dimethyl-2-oxobuttersäure (DOBS) sind in Tabelle 2 zusammengestellt.

Tabelle 2

| Beispiel Nr. | 6 | 7 | 8 | 9 | 10 | 11 | 12 | 13 | 14 | 15 |
|---|---|---|---|---|---|---|---|---|---|---|
| **Temp.** [°C] | 65 | 70 | 75 | 80 | 85 | 90 | 95 | 97,5 | 100 | 102,5 |
| **Zeit** [h] | 42 | 11 | 5,7 | 3,8 | 2,3 | 1,4 | 1,0 | 2,5 | 7,3 | 19 |
| **Ausbeute DOBS** [% d.Th.] | 61 | 73 | 76 | 81 | 77 | 83 | 91 | 92 | 90 | 94 |

### Beispiele 16 - 22

Es wird wie in Beispiel 1 gearbeitet und 0,1 mol (13,2 g) 3,3-Dimethyl-2-hydroxybuttersäure (DHBS) in 100 ml Natronlauge verschiedener Normalität an 1 g Aktivkohle mit 5 Gew.-% Palladiumgehalt bei Gegenwart von $5 \cdot 10^{-4}$ mol $Bi(NO_3)_3$ pro mol DHBS als Aktivator bei 95°C unter Normaldruck bis zur Aufnahme von ca. 1.300 ml Sauerstoff (ca. 0,535 mol $O_2$/mol DHBS) oxidiert. Die Ergebnisse hinsichtlich Reaktionszeit und polarographisch bestimmter Ausbeute an 3,3-Dimethyl-2-oxobuttersäure (DOBS) sind in Tabelle 3 zusammengestellt:

Beispiel 16 ist ein Vergleichsbeispiel, bei dem ohne überstöchiometrische Basenmenge gearbeitet wurde.

Tabelle 3

| Beispiel Nr. | 16 | 17 | 18 | 19 | 20 | 21 | 22 |
|---|---|---|---|---|---|---|---|
| mol **NaOH**/ / mol DHBS | 1 | 1,5 | 2 | 3 | 4 | 5 | 7,5 |
| **Reaktionszeit** [h] | >>6[*)] | 5 | 4,4 | 2 | 1,8 | 1 | 0,7 |
| **Ausbeute** DOBS [%d.Th] | nicht ermittelt | 86 | 86 | 90 | 87 | 91 | 82 |

*) Nach 6 Stunden sind erst ca. 2% (0,01 mol $O_2$/mol DHBS) aufgenommen und die $O_2$-Aufnahme kommt fast zum Stillstand.

### Patentansprüche

1. Verfahren zur Herstellung von 3,3-Dimethyl-2-oxobuttersäure bzw. deren Salzen durch übergangsmetallkatalysierte Oxidation von 3,3-Dimethyl-2-hydroxybuttersäure, **dadurch gekennzeichnet, dass** es in Gegenwart

   - von Sauerstoff,
   - Palladiumkatalysator,
   - Bismut und/oder Bismutverbindungen,
   - Wasser und
   - Base

   durchgeführt wird.

2. Verfahren nach Anspruch 1, **dadurch gekennzeichnet, dass** als Palladiumkatalysator metallisches Palladium oder auf einen Träger aufgebrachtes Palladium verwendet wird.

3. Verfahren nach einem oder mehreren der Ansprüche 1 und 2, **dadurch gekennzeichnet, dass** die Menge des Palladium-Katalysators so gewählt wird, dass das molare Verhältnis von Palladium zu eingesetzter 3,3-Dimethyl-2-hydroxy-buttersäure bzw. deren Salzen zwischen 1:5 und 1:20.000 beträgt.

**4.** Verfahren nach einem oder mehreren der Ansprüche 1 bis 3, **dadurch gekennzeichnet, dass** Aktivkohle als Träger verwendet wird.

**5.** Verfahren nach einem oder mehreren der Ansprüche 1 bis 4, **dadurch gekennzeichnet, dass** Bismut in elementarer Form und/oder in Form von Bismutverbindungen wie Oxid, Hydroxid, Oxidhydrat oder Salz von Wasserstoffsäuren wie Chlorid, Bromid, Jodid, Sulfid oder als Salz von anorganischen Sauerstoffsäuren wie Nitrat, Nitrit, Phosphit, Phosphat, Carbonat, Perchlorat oder als Salz von Sauerstoffsäuren, die von Übergangsmetallen abstammen, oder als Salz organischer Säuren oder als. Komplexverbindung oder als metallorganische Verbindung eingesetzt wird.

**6.** Verfahren nach einem oder mehreren der Ansprüche 1 bis 5, **dadurch gekennzeichnet, dass** die Menge an Bismut und/oder dessen Bismutverbindungen so gewählt wird, dass das molare Verhältnis von Bismut und/oder Bismutverbindung zu eingesetzter 3,3-Dimethyl-2-hydroxybuttersäure oder deren Salze $1 \cdot 10^{-6}$ bis $1 \cdot 10^{-1}$ beträgt.

**7.** Verfahren nach einem oder mehreren der Ansprüche 1 bis 6, **dadurch gekennzeichnet, dass** das Bismut und/oder die Bismutverbindungen in Kombination mit anderen Metallen und/oder Halbmetallen bzw. deren Verbindungen eingesetzt werden.

**8.** Verfahren nach einem oder mehreren der Ansprüche 1 bis 7, **dadurch gekennzeichnet, dass** die Konzentration von 3,3-Dimethyl-2-hydroxybuttersäure und deren Reaktionsprodukte in der Reaktionslösung 5 bis 30 Gew.-% beträgt.

**9.** Verfahren nach einem oder mehreren der Ansprüche 1 bis 8, **dadurch gekennzeichnet; dass** die Menge an Base so gewählt wird, dass auf ein Mol zu oxidierende 3,3-Dimethyl-2-hydroxybuttersäure insgesamt 1,1 bis 8 Äquivalente Base eingesetzt werden.

**10.** Verfahren nach einem oder mehreren der Ansprüche 1 bis 9, **dadurch gekennzeichnet, dass** als Base Alkali- oder Erdalkali-carbonate und -hydroxide, Mischungen davon oder entsprechende wässrige Lösungen eingesetzt werden.

**11.** Verfahren nach einem oder mehreren der Ansprüche 1 bis 10, **dadurch gekennzeichnet, dass** die Reaktion bei einem Druck von 0,1 bis 50 bar durchgeführt wird.

**12.** Verfahren nach einem oder mehreren der Ansprüche 1 bis 11, **dadurch gekennzeichnet, dass** die Reaktion bei einer Temperatur von 20 bis 150°C durchgeführt wird.

**13.** Verfahren nach einem oder mehreren der Ansprüche 1 bis 12, **dadurch gekennzeichnet, dass** die Reaktion bei einer Temperatur im Bereich von 60°C bis zum Siedepunkt des Reaktionsgemisches beim gewählten Reaktionsdruck durchgeführt wird.

**14.** Verfahren nach einem oder mehreren der Ansprüche 1 bis 13, **dadurch gekennzeichnet, dass** die Reaktion bei einer Sauerstoffaufnahme von 0,4 bis 0,6 mol Sauerstoff/mol 3,3-Dimethyl-2-hydroxybuttersäure abgebrochen wird.

**15.** Verfahren nach einem oder mehreren der Ansprüche 1 bis 14, **dadurch gekennzeichnet, dass** die erhaltene Lösung, welche die 3,3-Dimethyl-2-oxobuttersäure überwiegend in Form ihrer Salze enthält nach teilweiser oder vollständiger Neutralisation der überstöchiometrischen Base weiterverwendet wird.

**16.** Verfahren nach einem oder mehreren der Ansprüche 1 bis 15, **dadurch gekennzeichnet, dass** 3,3-Dimethyl-2-oxobuttersäure durch Ansäuern der Lösung mit einer Mineralsäure, wie z.B. Salz-, Schwefel- oder Phosphorsäure und anschließende Extraktion gewonnen wird.

**17.** Verwendung von 3,3-Dimethyl-2-oxobuttersäure, die gemäß einem Verfahren nach einem oder mehreren der Ansprüche 1 bis 16 gewonnen wurde, zur Herstellung von Agrochemikalien.

**18.** Verwendung nach Anspruch 17, **dadurch gekennzeichnet, dass** Metribuzin hergestellt wird.

**Europäisches Patentamt**

Nummer der Anmeldung

EP 03 01 7237

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | US 4 242 525 A (KIYOURA TADAMITSU) 30. Dezember 1980 (1980-12-30) *Zusammenfassung; Spalte 1, Zeilen 58-68; Spalten 2 und 3; die Beispiele und die Ansprüche* --- | 1-18 | C07C51/373 C07C59/185 |
| A | EP 0 011 207 A (MOBAY CHEMICAL CORP) 28. Mai 1980 (1980-05-28) *Zusammenfassung; Seiten 1-4; die Beispiele und die Ansprüche* --- | 1-18 | |
| A | US 5 091 568 A (JACKMAN DENNIS E) 25. Februar 1992 (1992-02-25) * das ganze Dokument * --- | 1-18 | |
| A | US 4 052 460 A (DICKORE KARLFRIED ET AL) 4. Oktober 1977 (1977-10-04) * das ganze Dokument * --- | 1-18 | |
| A | EP 1 097 917 A (KOREA RES INST OF CHEMICAL TEC) 9. Mai 2001 (2001-05-09) *Seiten 2-4 und die Ansprüche* --- | 1-18 | RECHERCHIERTE SACHGEBIETE (Int.Cl.7) C07C |
| A | EP 0 934 923 A (NIPPON KAYAKU KK) 11. August 1999 (1999-08-11) *Seiten 2 und 3; die Beispiele und die Ansprüche* ----- | 1-18 | |

Der vorliegende Recherchenbericht wurde für alle Patentansprüche erstellt

| Recherchenort | Abschlußdatum der Recherche | Prüfer |
|---|---|---|
| MÜNCHEN | 22. Januar 2004 | Lorenzo Varela, M.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**          EP 03 01 7237

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-01-2004

| Im Recherchenbericht angeführtes Patentdokument | Datum der Veröffentlichung | Mitglied(er) der Patentfamilie | Datum der Veröffentlichung |
|---|---|---|---|
| US 4242525 A | 30-12-1980 | JP 1236794 C | 31-10-1984 |
| | | JP 55098132 A | 25-07-1980 |
| | | JP 58042855 B | 22-09-1983 |
| | | JP 1348652 C | 28-11-1986 |
| | | JP 54138514 A | 27-10-1979 |
| | | JP 61015863 B | 26-04-1986 |
| | | JP 1348655 C | 28-11-1986 |
| | | JP 55033418 A | 08-03-1980 |
| | | JP 61016263 B | 28-04-1986 |
| | | CA 1101882 A1 | 26-05-1981 |
| | | CH 641140 A5 | 15-02-1984 |
| | | DE 2915395 A1 | 25-10-1979 |
| | | FR 2423472 A1 | 16-11-1979 |
| | | GB 2018773 A ,B | 24-10-1979 |
| | | IT 1120113 B | 19-03-1986 |
| | | NL 7902985 A ,B, | 19-10-1979 |
| EP 0011207 A | 28-05-1980 | AR 222669 A1 | 15-06-1981 |
| | | BR 7906994 A | 15-07-1980 |
| | | CA 1113936 A1 | 08-12-1981 |
| | | CS 209941 B2 | 31-12-1981 |
| | | DD 146945 A5 | 11-03-1981 |
| | | DE 2961465 D1 | 28-01-1982 |
| | | DK 477879 A ,B, | 14-05-1980 |
| | | EP 0011207 A1 | 28-05-1980 |
| | | HU 182906 B | 28-03-1984 |
| | | IL 58678 A | 30-11-1982 |
| | | JP 1430374 C | 09-03-1988 |
| | | JP 55066536 A | 20-05-1980 |
| | | JP 62039149 B | 21-08-1987 |
| | | US 4614822 A | 30-09-1986 |
| US 5091568 A | 25-02-1992 | BR 8700791 A | 15-12-1987 |
| | | CA 1291160 C | 22-10-1991 |
| US 4052460 A | 04-10-1977 | AT 346824 B | 27-11-1978 |
| | | AT 25277 A | 15-04-1978 |
| | | BE 850523 A1 | 19-07-1977 |
| | | BR 7700288 A | 20-09-1977 |
| | | CH 626039 A5 | 30-10-1981 |
| | | DE 2648300 A1 | 21-07-1977 |
| | | DK 20077 A | 21-07-1977 |
| | | ES 455148 A1 | 01-01-1978 |
| | | FR 2338922 A1 | 19-08-1977 |
| | | GB 1527472 A | 04-10-1978 |
| | | GB 1527473 A | 04-10-1978 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT
ÜBER DIE EUROPÄISCHE PATENTANMELDUNG NR.**

EP 03 01 7237

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-01-2004

| Im Recherchenbericht angeführtes Patentdokument | Datum der Veröffentlichung | Mitglied(er) der Patentfamilie | Datum der Veröffentlichung |
|---|---|---|---|
| US 4052460 A | | GB 1527474 A | 04-10-1978 |
| | | IL 51272 A | 30-11-1980 |
| | | IL 58657 A | 30-11-1980 |
| | | IT 1075057 B | 22-04-1985 |
| | | JP 1361704 C | 30-01-1987 |
| | | JP 52089610 A | 27-07-1977 |
| | | JP 61031093 B | 17-07-1986 |
| | | NL 7700446 A ,B, | 22-07-1977 |
| | | US 4564704 A | 14-01-1986 |
| | | US 4898974 A | 06-02-1990 |
| EP 1097917 A | 09-05-2001 | EP 1097917 A1 | 09-05-2001 |
| | | DE 69905369 D1 | 20-03-2003 |
| | | DE 69905369 T2 | 22-01-2004 |
| EP 0934923 A | 11-08-1999 | AU 728800 B2 | 18-01-2001 |
| | | AU 4220497 A | 02-04-1998 |
| | | BR 9711733 A | 24-08-1999 |
| | | CA 2266473 A1 | 19-03-1998 |
| | | DE 69720494 D1 | 08-05-2003 |
| | | EP 0934923 A1 | 11-08-1999 |
| | | HU 9903874 A2 | 28-03-2000 |
| | | US 6063955 A | 16-05-2000 |
| | | CN 1230171 A | 29-09-1999 |
| | | ES 2193398 T3 | 01-11-2003 |
| | | WO 9811057 A1 | 19-03-1998 |
| | | KR 2000030018 A | 25-05-2000 |
| | | RU 2169138 C2 | 20-06-2001 |

EPO FORM P0461

Für nähere Einzelheiten zu diesem Anhang : siehe Amtsblatt des Europäischen Patentamts, Nr.12/82